# EUROPÄISCHE PATENTSCHRIFT

(11) **EP 1 135 147 B1**
(45) Veröffentlichungstag und Bekanntmachung des Hinweises auf die Patenterteilung: **20.08.2003**
(21) Anmeldenummer: 99962088.3
(22) Anmeldetag: 24.11.1999
(51) Int. Cl.: A61K 35/78, A61K 9/20

(54) **VERFAHREN ZUR HERSTELLUNG VON ARZNEIMITTELN IN FESTER DARREICHUNGSFORM AUS PFLANZLICHEN EXTRAKTEN**
METHOD FOR PRODUCING MEDICAMENTS FROM PLANT EXTRACTS, IN A SOLID FORM OF ADMINISTRATION
PROCEDE DE PRODUCTION DE MEDICAMENTS SOUS FORME DE PRESENTATION SOLIDE A PARTIR D'EXTRAITS VEGETAUX

(30) Priorität: 24.11.1998 DE 19855287; 24.11.1999 DE 19957472
(43) Veröffentlichungstag der Anmeldung: 26.09.2001
(73) Patentinhaber: STEIGERWALD ARZNEIMITTELWERK GMBH, D-64295 Darmstadt (DE)
(72) Erfinder: KUPER, Willi, D-68649 Gross-Rohrheim (DE); MAIDONIS, Panagiotis, D-64293 Darmstadt (DE)
(74) Vertreter: Wablat, Wolfgang, Dr.Dr.
(86) Internationale Anmeldenummer: DE9903767
(87) Internationale Veröffentlichungsnummer: WO00030605

(56) Entgegenhaltungen:
- EP-A- 0 530 833
- EP-A- 0 867 177
- WO-A-96/37195
- DATABASE WPI Week 8120 Derwent Publications Ltd., London, GB; AN 1981-35188d XP002123427 & JP 56 030914 A (TSUMARA JUNTENDO KK), 28. März 1981 (1981-03-28)

## Beschreibung

Die Erfindung betrifft ein Verfahren zur Herstellung von Arzneimitteln in fester Darreichungsform aus pflanzlichen Extrakten, insbesondere Johanniskraut-Extrakt.

Arzneimittel in fester Form, wie Tabletten oder Kapseln, sind gegenüber der flüssigen Verabreichungsform bekanntermaßen insofern vorteilhaft, als sie in tieferen Abschnitten des Verdauungstraktes zur Wirkung kommen. Dadurch ermöglichen sie eine zielgerichtete Freisetzung der arzneilich wirksamen Bestandteile und somit eine besser zu steuernde Therapie. Die individuelle Behandlung des Patienten durch den Arzt kann über eine genaue Dosierung der Einzeldosis erfolgen. Zudem zeichnen sich die in fester Form vorliegenden Arzneimittel durch hohe Stabilität aus und können im wesentlichen problemlos verpackt, gelagert und transportiert werden.

Die Verabreichung von Tabletten oder Kapseln aus pflanzlichen Extrakten ist jedoch insofern nicht zufriedenstellend, als durch einen hohen Anteil an Hilfsstoffen in Kombination mit den in relativ geringen Anteilen vorliegenden arzneilich wirksamen pflanzlichen Extrakten zum einen das Resorptionsverhalten und zum anderen die Arzneiwirkstoffkonzentration am Resorptionsort bei derartigen Arzneimitteln gering ist. Darüber hinaus sind die Tabletten vergleichsweise groß, d.h. vom Patienten schwer einzunehmen.

Aus der DE 197 00 788 A1 ist bereits ein oral applizierbares Arzneimittel auf der Basis eines Johanniskrautextraktes bekannt, das in getrockneter Form als Pulver, Granulat, Kapsel oder Tablette verabreicht werden soll. Es wird jedoch keine technische Lehre vermittelt, wie eine mit pflanzlichem Extrakt hoch angereicherte Tablette tatsächlich hergestellt werden soll.

Die DE 196 39 375 A1 beschreibt ein Mistel-Trockenextrakt, dessen Verwendung ebenfalls als Pulver oder Granulat in einer Kapsel oder als Tablette oder Flüssigkeit erfolgen soll. Insbesondere ist in diesem Fall eine orale Applikation in calciumcarbonat- oder saccharidhaltiger Granulatform vorgesehen.

In der gleichen Darreichungsform ist aus der DE 196 27 376 A1 des weiteren die Verwendung eines Arzneimittels auf der Basis eines Artischocken-Extraktes bekannt.

Die oben genannten Arzneimittel sind alles in allem insofern nachteilig, als sie keine mit pflanzlichem Trokkenextrakt hoch angereicherte Verabreichungsform darstellen. Das heißt, bei einer ausreichend hohen Dosierung des Arzneimittels in Tablettenform müßten mehrere Tabletten oder eine nicht einnehmbar große Tablette verabreicht werden.

In der DE 36 16 054 A1 wird ein homöopathisches Arzneimittel beansprucht. Zu dessen Herstellung werden Triturationen von Schweineknochenmark, Schweinegelenkknorpel, Gelatine und Harpagophytine sowie einem äußerst hohen Anteil an Hilfsstoffen durch ein Sieb granuliert. Das Granulat wird mit weiteren Hilfsstoffen versetzt und in Tabletten verpreßt. Wegen des hohen Hilfsstoffanteils ist diese Art der Tablettenherstellung aus einem Granulat nicht auf die Herstellung von in oral verabreichungsfähiger Größe vorliegenden Tabletten aus Pflanzenextrakten, das heißt, von Tabletten mit einem möglichst geringen Hilfsstoffanteil und einem hohen Trockenextraktanteil, übertragbar.

Eine aus der DE 33 28 262 C 2 bekannte Arzneimittelzubereitung aus den Extrakten verschiedener Drogen kann entweder in flüssiger Form oder als Trockenextrakt in Pulver-, Granulat-, Tabletten- oder Kapselform verabreicht werden. Gemäß einer Ausführungsform wird das pulvrige Trockenextrakt unter Beimengung von Lactose und Magnesiumstearat zu Tabletten verpreßt, die anschließend zu einem Granulat als fertige Arzneimittelzubereitung zerkleinert werden. In einer zweiten Ausführungsform wird der pulvrige Trockenextrakt mit Cellulose und Magnesiumstearat vermischt und unmittelbar zu Tabletten verpreßt. Zwar werden hier feste Darreichungsformen mit einem hohen Anteil an pflanzlichen Extrakten angegeben, jedoch ist mit einer derart aus einem Trockenextrakt verpreßten Tablette die Einhaltung der von einem Arzneimittel geförderten Parameter, zum Beispiel Zerfallszeit oder Feuchtigkeitsschutz, nicht gewährleistet.

Die JP 78 13 347 und die JP 77 102 4416 beschreiben jeweils die Herstellung von aus pflanzlichen Extrakten unter Zumischung verschiedener Hilfsstoffe hergestellten Granulaten, ohne damit die Bedingungen für die industrielle Produktion von Tabletten mit einem hohen Trokkenextraktanteil, die zudem den sonstigen Anforderungen an ein herkömmliches Arzneimittel gerecht werden, eindeutig zu beanspruchen.

Der Erfindung liegt daher die Aufgabe zugrunde, ein Verfahren zur Herstellung von Fertigarzneimitteln in fester Darreichungsform mit einem pflanzlichen Extrakt als arzneilich wirksamem Bestandteil anzugeben, das ein galenisch stabiles, hochwirksames und am Freisetzungsort leicht lösliches und resorbierbares Medikament mit hohem Extraktanteil in vergleichweise geringer Tablettengröße zur Verfügung stellt.

Erfindungsgemäß wird die Aufgabe mit einem Verfahren gemäß den Merkmalen des Patentanspruches 1 gelöst.

Der Grundgedanke der Erfindung besteht mit anderen Worten in der Kompaktierung des Trockenextraktes zu Granulatpartikeln in Kombination mit einer dreischichtigen Maskierung des auf eine einheitliche Teilchengröße klassierten Granulats in einem Mischvorgang mit Titandioxid, Talkum und hochdispersem Siliciumdioxid. Die so ausgebildeten - verdichteten und gegebenenfalls mit weiteren Hilfsstoffen maskierten - Granulatteilchen werden anschließend in der üblichen Weise zu Tabletten oder Kapseln weiterverarbeitet.

Durch die Kombination der Schritte Kompaktieren, Klassieren auf eine einheitliche Korngröße und Maskieren in der angegebenen Weise wird ein für die industrielle Tabletten oder Kapselherstellung geeigneter Ausgangsstoff mit guten Fließeigenschaften geschaffen, der über einen äußerst hohen Extraktgehalt verfügt und mit einem entsprechend geringen Hilfsstoffanteil auskommt. Da der Extraktanteil in dem maskierten Kompaktat mindestens 65 Gew.-% beträgt, kann somit in einer Tablette üblicher Größe ein hochwirksames Medikament auf der Basis von pflanzlichen Extrakten zur Verfügung gestellt werden. Die bei der Maskierung der Granulatpartikel in der vorgegebenen Reihenfolge aufgebrachten Hilfsstoffe stehen der Wirksamkeit des Medikaments nicht entgegen, da die Granulatpartikel an den Außenflächen aufgrund der äußeren Schicht aus hochdispersem Siliciumdioxid nicht miteinander verschmelzen und somit nach der Einnahme leicht zerfallen und vollständig wirksam werden können.

Bei der dreistufigen Maskierung legt sich das vorzugsweise zuerst aufgebrachte Titandioxid unmittelbar an alle Oberflächenbereiche der Granulatpartikel an und bietet einen Feuchtigkeitsschutz für den üblicherweise hygroskopischen Extrakt. Mit dem anschließend auf die Titandioxidschicht aufgebrachten Talkum werden die verbleibenden Räume an der Granulatoberfläche gefüllt, um eine glatte, geschlossene Oberfläche zu erzielen. Schließlich dient das hochdisperse Siliciumdioxid auf der Außenfläche als Trennschicht zwischen den Granulatpartikeln in einer gepreßten Tablette. Die so maskierten Granulatpartikel zeichnen sich zudem durch eine für die Weiterverarbeitung vorteilhafte, besonders gute Fließfähigkeit aus.

Aus den Unteransprüchen sowie der nachfolgenden Beschreibung eines Ausführungsbeispiels ergeben sich weitere Merkmale und vorteilhafte Weiterbildungen der Erfindung.

Das erfindungsgemäße Verfahren zur Herstellung von Tabletten aus pflanzlichen Extrakten wird am Beispiel der Verwendung von Johanniskraut als arzneilich wirksamem Bestandteil zur Behandlung von Depressionen näher erläutert.

Ein aus grob geschnittenen Knospen und Blüten von Johanniskraut unter Verwendung eines Ethanol-Wasser-Gemisches erzeugter Flüssigextrakt wird durch Eindampfen zu einer dickflüssigen Masse aufkonzentriert und durch Sprühtrocknung in der Stufe A zu einem pulvrigen Trockenextrakt verarbeitet, dem zur Erleichterung des Trocknungsprozesses bestimmte Hilfsstoffe - hier Maltodextrin und hochdisperses Siliciumdioxid im Verhältnis 9 : 1 - in einem Anteil von 10 Gew.-% an dem pflanzlichen Trockenextrakt zugemischt wurden. Alternativ können auch andere Dextrine verwendet werden.

In der Stufe B wird der Trockenextrakt durch mechanische Verdichtung unter Zusatz des als Bindemittel dienenden Hilfsstoffs Lactose Monohydrat zu Granulat kompaktiert. In dieser Stufe können als alternative Hilfsstoffe Lactose-Derivate, Cellulose-Derivate, Stärke, Mannitol und Calciumcarbonat eingesetzt werden. In einem anschließenden Siebvorgang wird ein Kompaktat in einer einheitlichen Teilchengröße mit einem Hilfsstoffanteil von maximal 15 Gew.-% zur Verfügung gestellt.

Das in der Teilchengröße homogene, noch hygroskopische Kompaktat wird in der darauffolgenden Stufe C in einem ersten dreistufigen Mischvorgang zuerst mit Titandioxid, dann mit Talkum und anschließend mit hochdispersem Siliciumdioxid maskiert, um einen Feuchtigkeitsschutz und eine gute Fließfähigkeit sowie gute Zerfallseigenschaften der aus dem Kompaktat herzustellenden Tabletten zu bewirken. Die Homogenität des Kompaktats gewährleistet eine gleichmäßig dünne Beschichtung aller Teilchen. In einem zweiten und dritten Mischvorgang werden die Teilchen zusätzlich zunächst mit Lactose Monohydrat und Carboxymethyl-Cellulose-Natrium und anschließend mit Magnesiumstearat/Stearinsäure maskiert. Der Anteil an Hilfsstoffen beträgt jetzt höchstens 35 Gew.-%. Für Lactose Monohydrat können alternativ die oben erwähnten Hilfsstoffe und für Natriumcarboxymethylcellulose kann Natriumhydrogencarbonat, Calciumcarbonat, Stärke, Pektin, Magnesiumoxid, Kaliumbicarbonat und Kaliumcarbonat eingesetzt werden.

Die guten Fließeigenschaften der maskierten Kompaktatteilchen erlauben deren unmittelbare Weiterverarbeitung in einer Tablettenpresse (Stufe D). Die so hergestellten Tablettenkerne werden anschließend zum Licht-, Sauerstoff- und Feuchtigkeitsschutz mit einem Überzugsfilm versehen. Der Anteil an pflanzlichem Trockenextrakt beträgt danach mindestens 60 Gew.-%. Die so aus Pflanzenextrakten herstellbare Tablette ermöglicht bei guten Zerfallseigenschaften eine hohe und genaue Dosierung des Johanniskrautextraktes, und zwar bei geringer, d.h. einnehmbarer Tablettengröße.

Die Erfindung ist nicht auf das vorgehend geschilderte Ausführungsbeispiel beschränkt. Vielmehr sind im Rahmen des grundlegenden Erfindungsgedankens, das heißt der Herstellung eines aus Trockenextrakt erzeugten Kompaktats aus einheitlich großen, anschließend mit bestimmten Hilfsstoffen maskierten Teilchen, die zu Tabletten mit einem Extraktanteil zwischen 65 und 75 Gew.-% verpreßt werden können, verschiedene Modifikationen in bezug auf den Trockenextrakt und dessen Herstellung oder die verwendeten Hilfsstoffe denkbar.

## Patentansprüche

1. Verfahren zur Herstellung von Tabletten aus pflanzlichen Extrakten, insbesondere aus einem Johanniskrautextrakt, bei dem ein pulveriger pflanzlicher Trockenextrakt unter Zumischung von das Granulierverhalten verbesernden Hilfsstoffen zu einem Granulat kompaktiert wird und in der Größe homogene Granulatpartikel in einer dreistufigen Maskierung mit Titandioxid zum Feuchtigkeitsschutz, mit Talkum zur Oberflächenglättung und mit hochdispersen Siliziumdioxid als Trennmittel beschichtet werden und die so beschichteten Partikel anschließend zu Tabletten mit hohem Extraktanteil verpresst werden.

2. Verfahren nach Anspruch 1, **dadurch gekennzeichnet, daß** als Hilfsstoff bei der Kompaktierung des Trockenextrakts Lactose Monohydrat zugemischt wird.

3. Verfahren nach Anspruch 1, **dadurch gekennzeichnet, daß** das maskierte Kompaktat in weiteren Mischstufen mit zusätzlichen Hilfsstoffen behandelt wird.

4. Verfahren nach Anspruch 3, **dadurch gekennzeichnet, daß** als zusätzliche Hilfsstoffe zunächst Lactose Monohydrat/Carboxymethyl-Cellulose-Natrium und danach Magnesiumstearat/Stearinsäure verwendet werden.

5. Verfahren nach Anspruch 1 bis 4, **dadurch gekennzeichnet, daß** der Anteil an Hilfsstoffen im Verhältnis zu dem pflanzlichen Trockenextrakt 35 Gew.-% nicht überschreitet.

6. Verfahren nach Anspruch 1 bis 4, **dadurch gekennzeichnet, daß** der Hilfsstoffanteil bei der Trockenextraktherstellung 10 Gew.-%, bei der Kompaktierung 5 Gew.-% und bei der Maskierung 20 Gew.-% nicht überschreitet.

7. Verfahren nach Anspruch 1, **dadurch gekennzeichnet, daß** die Maskierung des Kompaktats mit Titandioxid, Talkum und hochdispersem Siliciumdioxid in der angegebenen Reihenfolge durchgeführt wird.

## Claims

1. A method for producing tablets from plant extracts, in particular, from a St. John's wort extract, wherein a powdery herbal dry extract is compacted to a granulate while adding adjuvants which improve granulating, and the granules of uniform particle size are masked in a three-step-process with titanium dioxide for providing moisture protection, talc for smoothing the surface, and highly dispersed silicon dioxide as parting agent, and the particles coated in this way are subsequently pressed to tablets with a high content of herbal extract.

2. The method according to claim 1 wherein lactose monohydrate is added as an adjuvant while compacting the dry extract.

3. The method according to claim 1 wherein the masked compacted particles are treated with additional adjuvants in subsequent mixing steps.

4. The method according to claim 3 wherein first lactose monohydrate and carboxymethyl cellulose sodium and then magnesium stearate and stearic acid are used as additional adjuvants.

5. The method according to claims 1 through 4 wherein the portion of adjuvants in relation to the dry plant extract does not exceed 35 percent by weight.

6. The method according to claims 1 through 4 wherein the portion of adjuvants does not exceed 10 percent by weight when producing the dry extract, does not exceed 5 percent by weight during compacting, and does not exceed 20 percent by weight during masking.

7. The method according to claim 1 wherein the compacted particles are masked with titanium dioxide, talc, and highly dispersed silicon dioxide in the specified order.

## Revendications

1. Procédé pour fabriquer des cachets à partir d'extraits végétaux, en particulier à partir d'un extrait de millepertuis, dans lequel :
- un extrait sec végétal en poudre est compacté en un granulé avec addition de substances auxiliaires qui améliorent le comportement de granulation,
- des particules de granulés homogènes quant à leur taille sont enduites, au cours d'un masquage à trois étapes, de dioxyde de titane pour la protection contre l'humidité, de talc pour le lissage surfacique, et de dioxyde de silicium fortement dispersé comme agent séparateur, et
- les particules ainsi enduites sont ensuite pressées pour former des cachets avec une haute teneur d'extraits.

2. Procédé selon la revendication 1, **caractérisé en ce que** du monohydrate de lactose est mélangé comme substance auxiliaire lors du compactage de l'extrait sec.

3. Procédé selon la revendication 1, **caractérisé en ce que** la masse compactée masquée est traitée avec des substances auxiliaires supplémentaires dans d'autres étapes de mélange.

4. Procédé selon la revendication 3, **caractérisé en ce qu'**on utilise tout d'abord du monohydrate de lactose/du sodium de cellulose carboxyméthylique comme substances auxiliaires supplémentaires, et **en ce qu'**on utilise ensuite du stéarate de magnésium/de l'acide stéarique.

5. Procédé selon l'une des revendications 1 à 4, **caractérisé en ce que** la part de substances auxiliaires ne dépasse pas 35 pour cent en poids par rapport à l'extrait sec végétal.

6. Procédé selon l'une des revendications 1 à 4, **caractérisé en ce que** la part de substances auxiliaires ne dépasse pas 10 pour cent en poids lors de la fabrication de l'extrait sec, 5 pour cent en poids lors du compactage, et 20 pour cent en poids lors du masquage.

7. Procédé selon la revendication 1, **caractérisé en ce que** le masquage de la masse compactée est exécuté avec du dioxyde de titane, du talc et du dioxyde de silicium fortement dispersé, dans l'ordre indiqué.
